# EUROPEAN PATENT APPLICATION

(11) **EP 0 537 399 A1**
(43) Date of publication of application: **21.04.1993**
(21) Application number: 91402767.7
(22) Date of filing: 16.10.1991
(51) Int. Cl.: C12N 15/82, C12N 15/55, C12N 9/22, C12N 15/31, A01N 63/02, C12N 5/10, A01H 5/00, A01H 1/02, C07K 15/00

(54) **A novel ribonuclease and its inhibitor**

(71) Applicant: PLANT GENETIC SYSTEMS, N.V., B-1040 Brussel (BE)
(72) Inventor: Nazarov, Victor Public Health Service, BETHESDA, Maryland 20892 (US); Botterman, Johan, B-9000 GENT (BE); Stanssens, Patrick, B-9830 St-Martens-Latem (BE); Sevcik, Joseph, 841 04 Bratislava (CS)
(74) Representative: Gutmann, Ernest

(57) **Abstract**

Novel RNase DNAs and their use in the disruption of the normal functioning, metabolism or development of cells of selected tissues or organs of eucaryotic organisms, particularly plants, transformed with the RNase DNAs. Also novel RNase-inhibitor DNAs and their use in restoring normal functioning, metabolism and development of cells disrupted by expression of the RNase DNAs.

## Description

This invention relates to a novel ribonuclease, containing only one disulfide bridge, and a novel inhibitor for the ribonuclease, both of which are obtainable from Streptomyces aureofaciens strain RO8/26, deposited at the DSM under accession no. 6691 on August 27, 1991.

This invention also relates to DNA coding for the novel ribonuclease and ribonuclease-inhibitor.

This invention further relates to the use of a first chimaeric gene comprising a DNA coding for a ribonuclease such as the novel ribonuclease of this invention, under the control of a promoter sequence adapted to provide expression of the ribonuclease-encoding DNA only in cells of selected organs and/or tissues of eucaryotic organisms, particularly plants, transformed with the chimaeric gene, so as to disrupt the metabolism selectively of such cells.

This invention still further relates to the use of a second chimaeric gene comprising a DNA coding for an ribonuclease-inhibitor such as the novel ribonuclease-inhibitor of this invention, under the control of a promoter sequence adapted to provide expression of the DNA encoding the ribonuclease-inhibitor at least in cells of eucaryotic organisms where the first chimaeric gene is expressed, so as to neutralize or inactivate the activity of the ribonuclease expressed in such cells.

This invention also relates to eucaryotic cells and organisms, particularly plants and their cells and seeds, transformed with the first and/or second chimaeric genes.

### Background of the Invention

Ribonucleases are secreted by many procaryotic and eucaryotic sources. One example of an eucaryotic ribonuclease is Rnase T1 which is produced by the fungus Aspergillus oryzae. A gene coding for this enzyme has been synthesized and expressed in Escherichia coli (Quaas et al (1988) Eur. J. Biochem. 173:617). Examples of secreted procaryotic ribonucleases include Rnase Sa from Streptomyces aureofaciens Type BMK and barnase from Bacillus amyloliquefaciens. The amino acid sequence of Rnase Sa has been determined (Shlyapnikov et al (1986) FEBS Letters 209:335) while the gene coding for barnase has been isolated from B. amyloliquefaciens (see Hartley (1989) TIBS 14:450 for a review).

It is also known that many organisms that secrete ribonucleases will also produce specific intracellular inhibitors for such ribonucleases. Presumably, the inhibitor serves as a defense against the toxic effects of intracellular ribonuclease activity. One example of such an inhibitor is barstar which is produced by B. amyloliquefaciens and forms a one-to-one noncovalent complex with barnase. The barstar gene has also been isolated (Hartley (1989) TIBS 14:450). An inhibitor for Rnase Sa from S. aureofaciens has also been isolated and characterized (Mucha et al (1983a) Biologia 38:1177; Mucha et al (1983b) Biologia 38:1185), but up to now, the corresponding gene has not been isolated.

Recently, the coding sequences of Rnase T1 and barnase have been placed under the control of a tapetum specific promoter and the resulting chimaeric genes have been used for the transformation of various species of plants such as Brassica napus. The intracellular expression of these ribonucleases was limited to the tapetum cell layer in the anthers of the transformed plants, and the plants were shown to be male sterile (European patent publication ("EP") 344 029; Mariani et al (1990) Nature 347:737).

A chimaeric gene consisting of the barstar gene under the control of the same tapetum specific promoter has also been used to transform plants. Crossing of male-sterile oilseed rape plants containing the chimaeric barnase gene with oilseed rape plants containing the chimaeric barstar gene resulted in progeny with fully restored fertility, proving that the barstar protein could inhibit the ribonuclease activity of barnase when genes encoding barnase and barstar are co-expressed in tapetum cells (EP 412 911; Mariani et al (1991) Proceedings of the CCIRC Rapeseed Congress, July 9-11, 1991, Saskatoon, Canada).

### Summary of the Invention

The present invention provides an improvement in known methods to disrupt significantly the normal metabolism, functioning and/or development of selected cells, especially the cells of one or more selected tissues and/or one or more selected organs ("target cells"), of a eucaryotic organism, preferably a plant, by providing in each cell of the organism, preferably in its nuclear genome, a first chimaeric gene comprising in the same transcriptional unit:
a first DNA sequence (a "RNase DNA"), preferably a RNase DNA from S. aureofaciens (a "sarnase DNA"), particularly a sarnase DNA from S. aureofaciens strain R08/26 (a "R08/26 sarnase DNA"); the RNase DNA coding for a protein (a "RNase") which has ribonuclease activity and which contains only one intramolecular disulfide bridge; the RNase preferably being an RNAse from S. aureofaciens (a "sarnase"), particularly a sarnase from S. aureofaciens strain R08/26 (a "R08/26 sarnase"), quite particularly the mature RO8/26 sarnase of SEQ ID no. 1;
a first promoter fused to the upstream (5') end of the RNase DNA and capable of directing expression of the Rnase DNA substantially only in the target cells of the organism; the RNase DNA being under the control of the first promoter; and
suitable transcription termination signals, including a polyadenylation signal, fused to the downstream (3') end of the RNase DNA.
The preferred target cells are: cells of tissue of male or female reproductive organs, seeds or embryos of plants, preferably anther, pollen or filament cells, particularly anther epidermal cells and/or tapetum cells, or style, stigma, ovule, septum, seed coat, endosperm, embryo axis and/or embryo cotyledon cells, whereby expression of the first chimaeric gene of this invention renders the plants male or female-sterile; and cells of plant tissue which has been invaded by a pathogenic organism such as a nematode, whereby expression of the first chimaeric gene of this invention protects the plant tissue from further damage by the pathogenic organism.

The present invention also provides an improvement in known methods to prevent or diminish the significant disruption of the normal metabolism, functioning or development of the target cells of a eucaryotic organism, preferably a plant, containing the first chimaeric gene in each of its cells, by providing in each of its cells, preferably in its nuclear genome, a second chimaeric gene comprising in the same transcriptional unit:
a second DNA sequence (a "RNase-inhibitor DNA"), preferably a RNase-inhibitor DNA from S. aureofaciens (a "sarstar DNA"), particularly a sarstar DNA from S. aureofaciens strain R08/26 (a "RO8/26 sarstar DNA"); the RNase-inhibitor DNA coding for a protein (a "RNase-inhibitor") which can inhibit the RNase activity of the RNase; the RNase-inhibitor preferably being a RNase-inhibitor from S. aureofaciens (a "sarstar"), particularly a sarstar from S. aureofaciens strain R08/26 (a "R08/26 sarstar");
a second promoter fused to the upstream end of the RNase-inhibitor DNA and capable of directing the expression of the Rnase-inhibitor DNA in at least the target cells; the Rnase-inhibitor DNA being under the control of the second promoter; and
suitable transcription termination signals, including a polyadenylation signal, fused to the downstream end of the RNase-inhibitor DNA.

In eucaryotic organisms, particularly plants, expression in the same cells of both the first and second chimaeric genes and their respective RNase DNA under the control of the first promoter and RNase-inhibitor DNA under the control of the second promoter can be especially useful. For instance, the progeny of two parents, the cells of one parent containing the RNAse DNA under the control of a first promoter which is only active in male reproductive organs and the cells of the other parent containing the RNase-inhibitor DNA under the control of a second promoter which is active in the same cells of the male reproductive organs as the first promoter, will have restored fertility. Under appropriate conditions, this effect can be used in the production of hybrid seed. Hence, the present invention further provides a cell (a "transformed cell") of an eucaryotic organism, particularly a plant, comprising the first chimaeric gene and/or the second chimaeric gene, especially as an insert in the genomic DNA of the cell; and eucaryotic organisms, particularly plants as well as plant cell cultures and seeds, consisting of such transformed cells.

The present invention still further provides: the S. aureofaciens strain R08/26 which was deposited at the DSM under accession number 6691; the R08/26 sarnase DNA, particularly the R08/26 sarnase DNA of SEQ ID no. 1; the R08/26 sarstar DNA; the R08/26 sarnase, particularly the mature R08/26 sarnase with the amino acid sequence of SEQ ID no. 1; the R08/26 sarstar; and the first and second chimaeric genes.

### Brief Description of the Drawings and the Sequences

In the following description of the invention and the examples, reference will be made to the following figures and sequences:

### FIGURES

- Figure 1:: Restriction map of 2.2 kb DNA fragment of Streptomyces aureofaciens strain R08/26 (DSM 6691) containing the R08/26 sarnase DNA of SEQ ID no. 1. The restriction sites under the line are important in the subcloning of the R08/26 sarnase DNA. The thick bar indicates the region whose sequence is given in SEQ ID no. 1.
- Figure 2:: Schematic representation of plasmid pTE4 (example 3). The most important features and restriction sites are shown. The numbers between brackets refer to cleavage sites in the sequence which is given in SEQ ID no. 3.

### SEQUENCE LISTING

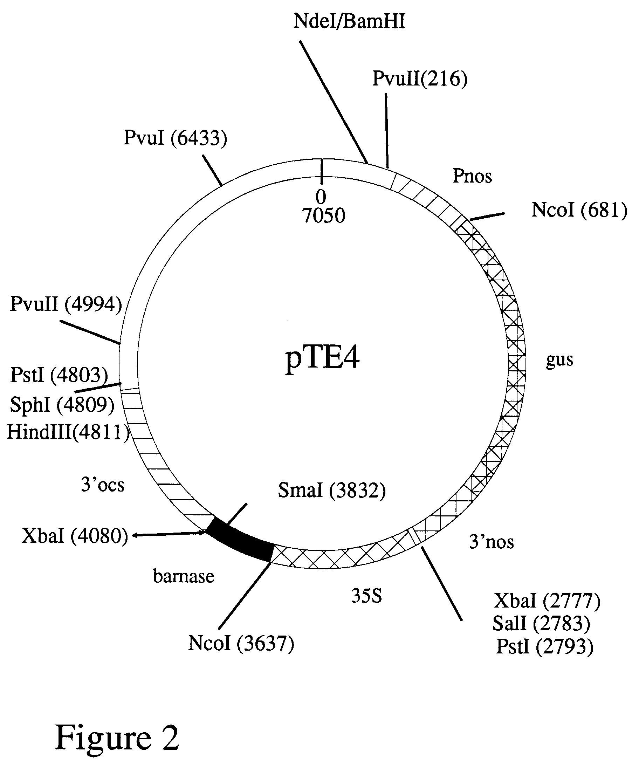

- SEQ ID no. 1:: nucleotide sequence comprising the novel R08/26 sarnase DNA from Streptomyces aureofaciens R08/26 (DSM 6691) and the corresponding amino acid sequence of the novel encoded sarnase.
- SEQ ID no. 2:: Nucleotide sequence of pMac5-19 (pMa5-19 and pMc5-19).
- SEQ ID no. 3:: Nucleotide sequence of pTE4 in Figure 2.

### Detailed Description of the Invention

The Streptomyces aureofaciens strain R08/26 of this invention was isolated by conventional means and deposited at the DSM under accession number 6691. This strain is characterized in that it secretes a novel sarnase-type ribonuclease. The cells of this strain are protected against the toxic effect of its sarnase by the intracellular expression of a novel sarstar-type ribonuclease-inhibitor. DNAs coding for the sarnase and sarstar of S. aureofaciens R08/26 can be isolated from this strain in a conventional manner. Equivalent sarnase DNAs and sarstar DNAs can be isolated from other strains of S. aureofaciens which are similar to S. aureofaciens R08/26 and which produce sarnases with only one disulfide bridge and corresponding inhibitory sarstars, such as S. aureofaciens Type BMK.

The RNase DNAs of this invention, coding for the RNases of this invention, can be isolated in a conventional manner. For example, the sarnase DNAs of this invention, coding for sarnases, can be obtained from S. aureofaciens strains and the like by conventional procedures, for instance as described in Example 1. For example, screening of a DNA library of total DNA of an S. aureofaciens strain can be carried out with an oligonucleotide corresponding to a sarnase which can be isolated from a S. aureofaciens, such as RNase Sa from S. aureofaciens type BMK (Shlyapnikov et al, supra), R08/26 sarnase from S. aureofaciens R08/26 having the sequence of SEQ ID no. 1, or an equivalent of RNase SA or R08/26 sarnase having substantially the same ribonuclease properties, particularly the capacity of being inhibited by a sarstar, such as the R08/26 sarstar.

A comparison of the expression and secretion in E.coli of RNase T1, which has two disulfide bridges, of a sarnase of this invention, which has only one disulfide bridge, and of barnase, which has no disulfide bridges, indicates that the sarnase has an intracellular toxicity (as measured by the growth of the bacterial culture) which is between that of RNase T1 and barnase, i.e., an intracellular toxicity greater than that of RNase T1 but less than that of barnase (see Example 2). Since disulfide bridges are formed with great difficulty in an intracellular environment and since proper disulfide bridge formation appears to be required for a protein to have ribonuclease activity, it is believed that the number of disulfide bridges is an important determining factor in the intracellular activity of a ribonuclease. In this regard, disulfide bridges are formed with difficulty in the cytoplasm of eucaryotic cells, such as plant cells, and proteins need to be translocated to the endoplasmic reticulum in order for disulfide bridges to be formed.

The RNase DNAs, particularly the sarnase DNAs, of this invention can provide an improvement in known processes for disrupting the otherwise normal metabolism, functioning or development of selected cells of eucaryotic organisms, particularly plants.

For instance, plants can be transformed with the first chimaeric gene of this invention, comprising an RNase DNA under the control of a first promoter which has a high specificity for cells of a specific tissue in male or female reproductive organs (i.e., for target cells of the plant). The resulting transgenic plants have dysfunctional male or female reproductive organs and are male sterile or female sterile due to the selective disruption of normal metabolism, functioning or development in the target cells. The use of DNAs coding for such first chimaeric genes and their RNases and first promoters for providing male- or female-sterility in plants has been extensively described in EP 344 029 and EP 412 006 which are incorporated herein by reference.

Plants can also be transformed with the first chimaeric gene of this invention comprising a RNase DNA under the control of a first promoter which is nematode-induced, i.e., a promoter whose action in controlling transcription of a DNA sequence is induced (e.g., stimulated) by infection of the plant by nematodes and preferably is induced selectively in cells of the plant's fixed feeding sites (e.g., giant cells, syncytia, nurse cells, and galls). The resulting transgenic plants will be resistant to nematode infections because of the plants' nematode induced breakdown of their fixed feeding sites which are essential for the survival of nematodes.

Preferably, the RNase DNAs, preferably sarnase DNAs, of this invention in the first chimaeric gene produce their encoded RNases, preferably sarnases, selectively in transformed target cells of an organism such as a plant so as to disrupt totally the cells' metabolism and thereby result in, for example, complete male- or female-sterility or nematode-resistance of the transformed plant. However in accordance with this invention, even if such RNases are also expressed to a significantly lesser degree in non-target cells of, for example, a plant (as a result of a relatively less selective first promoter in the first chimaeric gene), the RNases do not disrupt the metabolism, functioning or development of cells in other parts of the plant to an extent which could affect significantly the development or general agronomic performance of the plant.

The RNase-inhibitor DNAs of this invention, coding for the RNase-inhibitors of this invention, can also be isolated in a conventional manner. For example, the sarstar DNAs of this invention, coding for the sarstars, can be isolated from S. aureofaciens strains and the like by conventional procedures. For example, a sarstar can be purified from a bacterial strain according to the procedures described by Mucha et al (1983a) Biologia 38:1177, and its complete or N-terminal amino acid sequence can be determined. The gene coding for this sarstar can then be isolated from the strain as exemplified in Example 5. In this regard, an oligonucleotide corresponding to a characteristic part of the obtained amino acid sequence can be used to screen a bank of DNA fragments, obtained by digesting DNA of the strain with an appropriate restriction enzyme and cloned in a suitable vector in a suitable bacterial host such as E. coli. Positive clones can then be further analyzed, e.g., by sequencing. Alternatively, the DNA can be isolated directly by means of positive selection as exemplified in Examples 3 and 4. In this regard, DNA fragments of the strain can be cloned in a suitable bacterial host, such as E. coli or a Streptomyces strain that already contains the sarnase gene under the control of an inducible promoter (such as the tac promoter). Upon induction the cloned DNA fragments of surviving colonies can be further analyzed (e.g., by means of sequencing).

In accordance with this invention, the RNase-inhibitor DNAs coding for specific inhibitors, preferably the sarstars, particularly R08/26 sarstar, of the RNases, preferably the sarnases, particularly R08/26 sarnase, can be used to inhibit the activity of the specific corresponding RNases. For instance, plants can be transformed with the second chimaeric gene of this invention comprising a RNase-inhibitor DNA under the control of a second promoter which is active at least in cells of the same tissues of the male or female reproductive organs of the resulting restorer plants where the corresponding RNase is expressed in male- or female-sterile plants of the same species. Appropriate crossing of such restorer plants and sterile plants will result in progeny in which fertility is completely restored and can lead to the development of hybrid seed from which plants with fully restored fertility can be grown. The use of the RNase-inhibitor DNA for this purpose and the required procedures for transforming plants to make them fertility-restorer plants have been extensively described in EP 412 911 which is incorporated herein by reference.

The RNase DNAs of this invention, coding for RNases capable of forming only one intramolecular disulfide bridge (e.g., the sarnases), and the RNase-inhibitor DNAs of this invention, coding for specific inhibitors of such RNases (e.g., the sarstars), will be most useful in plants. However, these DNAs can be used to transform other eucaryotic organisms, in which it is desired either to disrupt the normal metabolism, functioning or development selectively of the cells of one or more specific tissues and/or organs of the organisms or to prevent such disruption.

The first and second chimaeric genes can also each comprise, or be fused to, a third chimaeric gene which contains, in the same transcriptional unit: a marker DNA encoding a marker RNA, protein or polypeptide which, when present at least in a specific tissue or specific cells of a transformed eucaryotic organism, particularly a plant, of this invention renders the organism easily separable or distinguishable from others which do not contain the marker RNA, protein or polypeptide at least in the specific tissue or specific cells; a third promoter fused to the 5' end of the marker DNA and capable of directing expression of the marker DNA at least in the specific tissue or specific cells; and suitable transcription termination signals, including a polyadenylation signal, fused to the 3' end of the marker DNA.

The use of a particular marker DNA in each third chimaeric gene also is not believed critical, and if desired, the marker DNAs associated with each of the first and second chimaeric genes can be the same or different. Examples of suitable marker DNAs for plants are disclosed in EP 344 029, EP 412 006 and EP 412 911. In this regard, the marker DNAs can encode proteins or polypeptides inhibiting or neutralizing the activity of herbicides such as: the sfr gene and the sfrv gene encoding enzymes conferring resistance to glutamine synthetase inhibitors such as Bialophos and phosphinotricine as described in European patent application ("EPA") 87400544.0; and genes encoding modified target enzymes for certain herbicides that have a lower affinity for the herbicides than naturally produced endogenous enzymes, such as a modified glutamine synthetase as target for phosphinotricine as described in EP 240 792 and a modified 5-enolpyruvylshikimate-3 phosphate synthase as a target for glyphosate as described in EP 218 571.

In the first and second chimaeric genes of this invention, the use of particular first and/or second promoters is not believed critical, and if desired the first and second promoters can be the same. Examples of male organ-specific first and/or second plant promoters are the PTA29 promoter, the PTA26 promoter and the PTA13 promoter described in EP 344 029, which have been isolated from tobacco and are tapetum-specific promoters, and promoters of the plant genes encoding tapetum-specific mRNAs hybridizable to the genes TA29, TA26 or TA13 of EP 344 029, from which the PTA29, PTA26 and PTA13 promoters have been isolated, as well as the tapetum-specific promoter of PCT publication WO 90/08825. Examples of female organ-specific first and second plant promoters are described in EP 412 006.

In many cases, the second promoter (which controls the RNase-inhibitor DNA) and/or the third promoter (which controls the marker DNA) are preferably each a constitutive promoter whereby the RNase inhibitor DNA and/or the marker DNA will be expressed in the entire eucaryotic organism, e.g., plant. The selection of particular second or third promoters would then not be considered critical. Examples of suitable second and third constitutive plant promoters are disclosed in EP 344 029, EP 410 006 and EP 412 911, such as the strong constitutive 35S promoter (Odell et al (1985) Nature 313:810-812), 35S'3 promoter (Hull and Howell (1987) Virology 86:482-493), promoter of the nopaline synthetase gene ("PNOS") of the Ti-plasmid (Herrera-Estrella (1983) Nature 303:209-213) or promoter of the octopine synthase gene ("POCS" [De Greve et al (1982) J. Mol. Appl. Genet. 1 (6):499-511)].

In the chimaeric genes of this invention, 3' transcription termination signals can be selected from among those which are capable of providing correct transcription termination and polyadenylation of mRNA in plant cells. The transcription termination signals can be the natural ones of the RNase DNA or RNase-inhibitor DNA or heterologous. Examples of heterologous transcription termination signals are those of the octopine synthase gene (Gielen et al (1984) EMBO J. 3:835-845) and the T-DNA gene 7 (Velten and Schell (1985) Nucleic Acids Research ("NAR") 13:6981-6998).

Eucaryotic organisms can be transformed with the chimaeric genes of this invention in a conventionnal manner. In this regard, the cell of a plant, particularly a plant capable of being infected by Agrobacterium, can be suitably transformed using a vector that is a disarmed Ti-plasmid containing the first and third chimaeric genes of this invention or the second and third chimaeric genes of this invention and carried by Agrobacterium. This transformation can be carried out using the procedures described, for example, in EP 116 718 and EP 270 822. Preferred Ti-plasmid vectors contain one or more of the chimaeric genes of this invention between the border sequences, or at least located to the left of the right border sequence, of the T-DNA of the Ti-plasmid. Of course, other types of vectors can be used to transform the plant cell, using procedures such as direct gene transfer (as described, for example, in EP 233 247), pollen mediated transformation (as described, for example, in EP 270 356, PCT publication WO 85/01856, and US patent 4,684,611), plant RNA virus-mediated transformation (as described, for example, in EP 067 553 and US patent 4,407,956) and liposome-mediated transformation (as described, for example, in US patent 4,536,475). If the plant to be transformed is corn, it is preferred that more recently developed methods be used such as, for example, the method described for certain lines of corn by: Fromm et al (1990) Bio/Technology 8:833; Gordon-Kamm et al (1990), Bio/Technology 2:603; and Gould et al (1991) Plant Physiol. 95:426. If the plant to be transformed is rice, it is preferred that recently developed methods be used such as, for example, the method described for certain lines of rice by: Shimamoto et al (1989) Nature 338:274; Datta et al (1990) Bio/Technology 8:736; and Hayashimoto et al (1990) Plant Physiol. 93:857.

A transformed plant cell of this invention can be regenerated into a mature plant, and the resulting transformed plant can be used in a conventional breeding scheme to produce more transformed plants with the same characteristics or to introduce the first and/or second chimaeric genes of this invention in other varieties of the same or related plant species. Seeds obtained from the transformed plants contain each of the chimaeric genes of this invention as a stable genomic insert.

In the Examples which follow, all experimental procedures for manipulating recombinant DNA were carried out, unless otherwise indicated, by the standardized procedures described in Sambrook et al (1990) Molecular Cloning: a Laboratory Manual, Cold Spring Harbor Laboratory. Oligonucleotides were designed according to the general rules outlined by Kramer and Fritz (1968) Methods in Enzymology 154:350 and synthesized by the phosphoramidite method described by Beaucage and Caruthers (1981) Tetrahedron Letters 22:1859 on an applied Biosystems 380A DNA synthesizer (Applied Biosystems B.V., Maarssen, Netherlands).

The following bacterial strains and plasmids, used in the examples, are available from the Deutsche Sammlung für Mikroorganismen und Zellkulturen ("DSM"), Mascheroder Weg 1B, Braunschweig, Germany:

| Bacterial strain | plasmid | DSM No. | Date of Deposit |
|---|---|---|---|
| E.coli WK6 | pMa5-8 | DSM 4567 | May 3, 1988 |
| E.coli WK6 | pMc5-8 | DSM 4566 | May 3, 1988 |
| E.coli MC1061 | pMB3 | DSM 4470 | March 22, 1988 |
| E.coli MC1061 | pTTM6 | DSM 4468 | March 21, 1988 |
| E.coli K514 | pGSJ260 | DSM 3606 | December 12, 1985 |
| E.coli MC1061(λ) | pEcoR251 | DSM 4711 | July 13, 1988 |
| Streptomyces aureofaciens R08/26 | - | DSM 6691 | August 27, 1991 |

### Examples

### Example 1: Isolation and cloning of the R08/26 sarnase DNA from Streptomyces aureofaciens strain R08/26 (DSM 6691)

Total DNA of Streptomyces aureofaciens R08/26 (DSM 6691) was digested with PstI, and DNA fragments of 2.0 to 2.4 kb in length were isolated on LGT (low gelling temperature) agarose. Individual fragments were inserted in the PstI site of pEcoR252, and the resulting plasmids were transformed in E.coli MC1061 which were grown on LB medium supplemented with 100 mg/l ampicillin. Plasmid pEcoR252 was derived from pEcoR251 (DSM 4711) by exchanging a 253 bp BglII-PvuI fragment of the β-lactamase gene of pEcoR251 with the corresponding fragment of pUC19 (Yanisch-Perron et al (1985) Gene 33:103).

300 clones were screened by means of colony hybridization, using Hybond N+ nylon membranes (Amersham Intl., PLC, Buckinghamshire, England) and a washing temperature of 30°C, with the following oligonucleotide probe:
TAT GGI TAT TAT CAT GAA TAT AC.

The oligonucleotide was constructed on the basis of the amino acids 49 to 56 (Tyr-Gly-Tyr-Tyr-His-Glu-Tyr-Thr) of the known amino acid sequence of the sarnase, RNase Sa, from S. aureofaciens Type BMK (Shlyapnikov et al, supra).

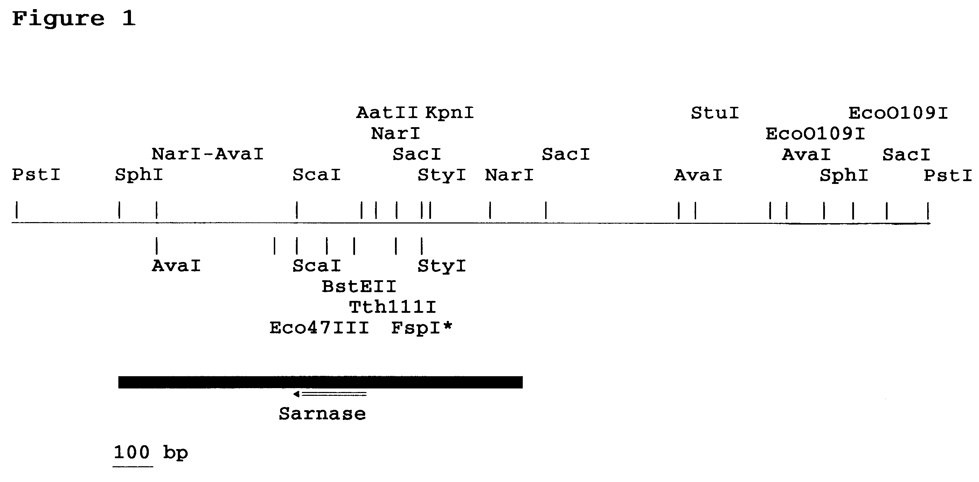

Three positive clones were identified which were all shown to carry an identical 2.2 kb insert in the same orientation. This clone was named pEcoR252/P1. The restriction map of the insert was determined (see Fig. 1), and the insert was sequenced according to Maxam and Gilbert (1980) Methods in Enzymology 65:499. The sequence of a 977 bp fragment containing the sequence coding for the R08/26 sarnase is given in SEQ ID no. 1.

The expression vector pMa5-19 was derived from pMa5-8 (DSM 4567) as follows. The EcoRI-HindIII fragment of plasmid pMT416, containing the barnase gene, as described by Hartley (1988) J. Mol. Biol. 202:913, was cloned between the EcoRI and HindIII sites of pMa5-8. The sequence at the transition of the PhoA signal sequence and the barnase coding sequence was changed from GCC GCA CAG to GCG GTA CCG by site-directed mutagenesis, thus introducing a KpnI site. Subsequently the GTG initiation codon of the PhoA signal sequence was changed to ATG by site-directed mutagenesis. Finally, the sequence between the KpnI site and the HindIII site was replaced with the KpnI-HindIII fragment of pUC19 containing the polylinker of pUC19.

Using the analogous procedure, plasmid pMc5-19 is derived from pMc5-8 (DSM 4566) and from pMa5-19 (Stanssens et al (1989) NAR 17:4441). The sequence of the plasmid, pMac5-19, is given in SEQ ID. no. 2.

pMa5-19 and pMc5-19 contain the tac-promoter and the PhoA signal sequence from pMT416, flanked at the 3' end with most of the polylinker of pUC19. pMa5-19 and pMc5-19, like pMa5-8 and pMc5-8, can be introduced in E.coli WK6 (Zell and Fritz (1987) EMBO J. 6:1809), and the bacteria can then be grown in minimal medium supplemented with 100 mg/l ampicillin for pMa5-19 or with 25 mg/1 chloramphenicol for pMc5-19. In E. coli strains that overproduce the lac repressor, such as strain WK6, the expression of a gene under the control of the tac promoter can be regulated. While normally repressed, gene expression can be induced by addition of a commonly used inducer of the lac operon, IPTG (isopropyl-β-d-thiogalactopyranoside). Plasmids pMa5-8 and pMc5-8 and derivatives thereof, such as pMa5-19 and pMc5-19, can be used for site-directed mutagenesis by a gapped duplex DNA method using alternating selectable markers (Stanssens et al (1989) NAR 17:4441; EPA 87402348.4).

Because the cloning of the complete gene encoding R08/26 sarnase in the expression vectors pMa5-19 or pMc5-19 was expected to have toxic effects on the host cell, it was decided to prepare subclones of two overlapping parts of the R08/26 sarnase gene. Where required during the cloning procedures, the orientation of cloned inserts was checked by means of restriction mapping or sequencing. To clone the proximal part of the R08/26 sarnase gene, plasmid pEcoR252/P1 was cleaved with StyI, filled in with the large fragment of DNA Polymerase I of E.coli (Klenow), and cleaved again with ScaI, and the 335 bp fragment was cloned in the SmaI site of pMc5-19, yielding plasmid pMc5-19/Sty-Sca. For further manipulation, a FspI site was created immediately upstream the presumed coding sequence (starting with Ala-Asp) of the mature R08/26 sarnase. The gapped duplex DNA was constructed from the single stranded pMa5-19/Sty-Sca and the large KpnI-Tth111I fragment of pMc5-19/Sty-Sca. For mutagenesis, use was made of the following oligonucleotide:
GCCGGGTCTGCGCACGTCCAGGCG
yielding plasmid pMc5-19/Mut-Fsp. Finally, a deletion was made by ligating the 2274 bp FspI fragment of pMc5-19/Mut-Fsp and the 1970 bp KpnI (filled-in with Klenow)-FspI fragment of pMa5-19/Tth-Ava (see below), yielding pMa5-19/deltaKF.

To clone the distal part of the R08/26 sarnase gene, plasmid pEcoR252/P1 was cleaved with Tth111I, filled in with Klenow, and cleaved again with AvaI, and the 347 bp fragment was cloned in the SmaI site of pMc5-19. A 583 bp EcoRI-HindIII fragment was isolated from DNA prepared from the mixture of transformants and cloned between the EcoRI and HindIII sites of pMa5-8, yielding plasmid pMa5-19/Tth-Ava. The 3' end of the distal part of the R08/26 sarnase gene was then deleted by cleaving pMa5-19/Tth-Ava with SphI, filling in with Klenow, cleaving with Eco47III and religating, yielding pMa5-19/deltaES.

Both parts of the R08/26 sarnase gene were then joined as follows. The large EcoRI-BstEII fragment of pMa5-19/deltaES was ligated to the 370 bp EcoRI-BstEII fragment of pMa5-19/deltaKF, yielding pMa5-19/Sarnasel. The large EcoRI-BstEII fragment of pMa5-19/deltaES was also ligated to the 470 bp EcoRI-BstEII fragment of pMa5-19/Mut-Fsp, yielding pMa5-19/Sarnpgs1. pMa5-19/Sarnasel, containing the mature R08/26 sarnase coding sequence immediately behind the tac-PhoA region, can be used for expression of R08/26 sarnase in E. coli. pMa5-19/Sarnpgs1 contains the mature R08/26 sarnase sequence as a 306 bp FspI-XbaI or as a 298 bp FspI-HindIII fragment. These fragments can therefore be used for the construction of gene cassettes for plant transformation (see Example 7).

Surprisingly, the amino acid sequence of the R08/26 sarnase isolated from S. aerofaciens R08/26 is quite different from that of the sarnase, RNase Sa, isolated from S. aureofaciens (Shlyapnikov et al (1986) FEBS Letters 209:335). The differences between the two sarnases could be due to their bacterial sources. In this regard, the R08/26 sarnase codes for a RNase with a quite different amino acid sequence having only about 50% homology to the amino acid sequence of RNase Sa. R08/26 sarnase has only two cysteine residues which are homologous to the cysteine residues in RNase Sa that are involved in disulfide bridge formation (Sevcik et al (1991) Acta Cryst. B47:240). It is highly likely that the two cysteines of R08/26 sarnase also form its disulfide bridge.

### Example 2: Comparison of ribonuclease expression on viability of E.coli.

The expression and secretion of ribonucleases in transformed E. coli WK6 and the effects of the ribonucleases on the viability of the host cells were compared in host cells transformed with pMa5-19/Sarnase of Example 1 and the similar constructs pMa5-RT1 and pMa5-Barnase containing respectively the Rnase T1 gene from Aspergillus oryzae and barnase gene from B. amyloliquefaciens.

pMa5-RT1 was derived from pMa5-8 and contains the tac-PhoA-RnaseT1 gene cassette. Its construction has been described in Steyaert et al (1990) Biochemistry 29:9064.

pMa5-Barnase was constructed by cloning the EcoRI-HindIII fragment of pMT416 (Hartley (1988) J.Mol.Biol. 202:913) between the EcoRI and HindIII sites of pMa5-8. This construct also contains an expressible barstar gene from B. amyloliquefaciens.

The effects of pMa5-19/Sarnasel, pMa5-RT1 and pMa5-Barnase in E.coli WK6 on the growth curves of uninduced and induced bacteria were ascertained according to Steyaert et al (1990), supra, and the results are qualitatively summarized in the following table.

| Plasmid | Expressed genes | cell growth repressed conditions | cell growth after IPTG induction |
|---|---|---|---|
| pMa5-19 | None | +++ | +++ |
| pMa5-RT1 | RNase T1 | +++ | ++ |
| pMa5-19/Sarnasel | R08/26 Sarnase | + | - |
| pMa5-Barnase | Barnase/Barstar * | + | - |

| | | | |
|---|---|---|---|
| * Expression of barnase alone (without barstar) is lethal even under repressed conditions (Hartley (1989) TIBS 14:450). | | | |

These results indicate that the effect of expression of the R08/26 sarnase gene in E. coli is intermediate with respect to that observed with RNase T1 and barnase. It is believed that this is due to the presence of a single disulfide bridge in the R08/26 sarnase. Disulfide bridges are formed only with difficulty in the reducing intracellular environment while, to be active, enzymes require such bridges to be properly formed. If no disulfide bridges are present (as in barnase), the enzyme is always active, and its presence in the cell is lethal. If two disulfide bridges are present (as in RNase T1), almost no active enzyme is produced intracellularly, even after induction. With only one disulfide bridge, as in a sarnase, a larger proportion of active enzyme is produced which is reflected in the reduced viability of the repressed host cells and the complete loss of viability after induction.

### Example 3: Cloning of the R08/26 sarstar gene from S. aureofaciens R08/26 by positive selection from E. coli

The BamHI (filled-in with Klenow)-EcoRI fragment of plasmid pEcoR252 (from Example 1), containing the gene coding for the EcoRI restriction endonuclease under control of the P_{R} promoter, is ligated to the large BssHI (filled-in with Klenow)-EcoRI fragment of plasmid pMa5-19/sarnpgs1 (from Example 1) yielding plasmid pMa5-19/sarnpgs2.

The total DNA obtained from S. aureofaciens R08/26 is partially digested with Sau3AI. Fragments with sizes ranging from 4 kb to 10 kb are isolated using LGT agarose electrophoresis and are ligated to pMa5-19/Sarnpgs2 linearized with BglII. The ligation mixture is used to transform competent cells of E.coli WK6. Transformants are grown on LB agar plates supplemented with IPTG (final concentration 0.1 mM). Positive clones, which are thought to express the gene coding for the R08/26 sarnase inhibitor (R08/26 sarstar) using the P_{R} promoter and its own translational signals, are further analyzed using restriction analysis, sequencing and inhibitor activity estimation (Mucha et al (1983a) Biologia 38:1177) to isolate the R08/26 sarstar DNA.

### Example 4: Cloning of the R08/26 sarstar gene from S. aureofaciens R08/26 by positive selection in Streptomyces lividans

Total DNA from S. aureofaciens R08/26 is partially digested with Sau3AI. Fragments with sizes ranging from 4 kb to 10 kb are isolated using LGT agarose electrophoresis and are ligated to pKJ4/P1 linearized with BglII or BamHI. pKJ4/P1 is a shuttle vector for E. coli and Streptomyces (Nazarov and Godany (1991) In 'Metabolism and enzymology of nucleic acids including gene and protein engineering' - Balan J., ed. - Czechoslovakian Academy of Sciences, Proceedings of a symposium held in Bratislava on November 26-30, 1990, pp. 117-123). The ligation mixture is used to transform competent cells of E. coli MC1061. Recombinant plasmids are isolated from transformants and used to transform Streptomyces lividans 66 protoplasts (see Thompson et al (1987) EMBO J 6:2519). Plasmids from positive clones are analyzed using direct inhibitor activity estimation. After transfer into E. coli, the plasmids are further characterized by means of restriction analysis and sequencing to isolate the R08/26 sarstar DNA.

### Example 5: Cloning of the R08/26 sarstar gene from S. aureofaciens R08/26 by oligonucleotide screening of a genomic library

R08/26 sarstar is purified from S. aureofaciens R08/26 according to the procedures described by Mucha et al (1983a) supra. The N-terminal sequence of the R08/26 sarstar is then determined using procedures as described by Bauw et al (1987) Proc. Natl. Acad. Sci. USA ("PNAS") 84:4806. On the basis of the N-terminal amino acid sequence, a number of oligonucleotides encoding the sequence are then designed and synthesized to screen, by means of nucleic acid hybridization, a genomic DNA library of S. aureofaciens R08/26. The genomic library is obtained by digestion of total DNA of the strain with Sau3AI and cloning of the DNA fragments in pUC19 in E.coli MC1061. Positive clones are then further analyzed by restriction enzyme mapping and DNA sequencing to isolate the R08/26 sarstar DNA.

### Example 6: Comparison of RNase T1, barnase and R08/26 sarnase as cytotoxic gene products in plant cells.

Expression of the barnase gene from B. amyloliquefaciens and the RNase T1 gene from A. oryzae under the control of a plant promoter specifically active in the tapetal cell layer has shown that the expression of the corresponding ribonuclease causes male sterility in plants (Mariani et al, supra).

To evaluate the efficacy of RNase T1, barnase and R08/26 sarnase in plant cells, analyses are performed based on transient expression assays (Denecke et al (1989) Methods Mol. Cell Biol. 1:19). A DNA encoding each ribonuclease is placed under the control of the constitutive CaMV 35S promoter. To measure the lethal effect of the expression of each ribonuclease, a marker gene construct, together with the ribonuclease-encoding DNA, is introduced in plant protoplasts. The marker gene is gus (Jefferson et al (1987) EMBO J. 6:3901). The expression of each ribonuclease and its impact on cell metabolism is indirectly measured by the gus expression profile. The presence or absence of the ribonuclease in the plant cell is modulated by using plasmid DNA linearized respectively outside or inside the coding region of the ribonuclease-encoding DNA. Upon electroporation of the linearized DNA, the gus expression profile is followed as a function of time and gene dosage in order to compare the effects of the different ribonucleases.

Small plasmids, pTE4, are used in order to reduce the impact of plasmid size on transient expression assays, and each plasmid contains a chimeric gus gene construct and a chimeric ribonuclease gene construct. pTE4 is shown in Fig. 2, and its complete sequence is shown in SEQ ID no. 3. pTE4 is a pUC18 derived plasmid containing two chimaeric genes cloned in the polylinker. One of these genes is the pnos-gus-3'nos gene which consists of a pnos promoter fragment (De Picker et al (1982) J.Mol.Appl.Genet. 1:561), the GUS coding sequence (Jefferson et al, supra) and the 3' untranslated end of nopaline synthase (Gielen et al (1984) EMBO J 3:835). The other chimeric gene is a CaMV 35S-barnase-3'ocs gene construct which consists of a CaMV 35S promoter fragment (EP 359 617), the barnase coding region and the 3' untranslated end of octopine synthase (Ingelbrecht et al (1989) The Plant Cell 1:671).

pTE4 can only be propagated in a host strain carrying an active barstar gene. To this end, the strain WK6(pMc5BS) is used. Plasmid pMc5BS contains the barstar gene under the control of the tac promoter and is constructed by cloning the EcoRI-HindIII fragment of pMT416 (Hartley, supra) in pMc5-8 (DSM 4566).

Then, the sequence, starting with the initiation codon of the phoA signal sequence and ending with the last nucleotide before the translation initiation codon of the barstar coding region, is deleted by looping-out mutagenesis according to the general procedures described by Sollazzo et al (1985) Gene 37:199. The availability of an ampicillin resistance gene on pTE4 and a chloramphenicol resistance gene on pMc5BS permits the strains to be kept stable on plates provided with the two antibiotics.

To obtain comparable plasmids with the RNase T1 and R08/26 sarnase coding region, the following constructs are made :
- pCV10 : the HindIII (filled-in with Klenow.) - NcoI restriction fragment of pTE4 containing barnase-3'nos is replaced by the EcoRI (filled-in with Klenow.) - NcoI fragment containing RNase T1-3'nos from pTTM6 (DSM 4468).
- pCV9 : the HindIII-NcoI restriction fragment of pTE4 containing barnase-3'nos is replaced by the HindIII-NcoI fragment containing R08/26 sarnase-3'nos from pCO50 (see Example 7).

The plasmids pTE4, pCV9 and pCV10 are linearized in two different ways: 1) by cleavage of a unique site within the coding region of the ribonuclease-encoding DNA (SmaI in pTE4, BamHI in pCV9 and BstEII in pCV10), and 2) by cleavage of a unique site outside of the chimeric gene constructs (PvuI in all cases). Linearized plasmid DNA is electroporated in mesophyll protoplasts prepared from leaves of SR1, and GUS activities are measured as described by Denecke et al, supra. In all cases in which an intact ribonuclease DNA is introduced in plant protoplasts together with an intact gus gene, the GUS activity profile, as a function of time, is significantly quenched. However, as compared to the control situations in which protoplasts are electroporated with plasmid DNA linearized within the ribonuclease coding region (so that only an intact gus gene is present), GUS activity is most quenched by expression of barnase (pTE4) and less quenched by expression of RNase T1 (pCV10). Expression of R08/26 sarnase (pCV9) results in a level of quenching of GUS activity that is intermediate with respect to the expression of barnase and the expression of RNase T1. This indicates that the toxic effect of sarnase expression in plant cells is intermediate to those of barnase expression and RNase T1 expression. The differences in toxic effects among the three ribonuclease-encoding genes may be due to differences in efficiency of transcription/translation of the respective genes and/or of post-translational processes that have an effect on enzyme activity (e.g. proper folding and disulfide bridge formation). However, as the three ribonuclease-encoding DNAs are under the control of identical regulatory signals, it is believed that the differences in enzyme activity (as measured by the degree of GUS activity quenching) are most likely due to differences in the efficiency of protein folding and disulfide bridge formation.

### Example 7: Construction of T-DNA vectors, each containing the R08/26 sarnase coding region or the R08/26 sarstar coding region under the control of the tapetum specific promoter, PTA29.

A T-DNA vector carrying the R08/26 sarnase DNA of Example 1 under the control of the tapetum specific TA29 promoter, similar to the RNase T1-encoding pTTM6A and the barnase-encoding pTTM8 of EP 344 029, is constructed. In a first step, the R08/26 sarnase DNA is provided with a fragment for transcription termination and polyadenylation (3'ocs) as follows: a HindIII-EcoRI fragment from pMa5-19/sarnpgs1 is cloned in pOCS1 digested with SmaI and EcoRI, yielding pCO49. Plasmid pOCS1 is constructed by ligating the 0.7 kbp PvuII fragment comprising the 3' untranslated end of the octopine synthase gene of the T-DNA from Agrobacterium tumefaciens, as described by Ingelbrecht et al (1989) The Plant Cell 1:671, in the HindII site of plasmid pUC19 (Yanisch-Perron et al (1985) Gene 33:103). In a second step, a chimeric PTA29-R08/26 sarnase-3'ocs gene construct is made by combining i) a NcoI (filled-in with Klenow)-EcoRI fragment of pMB3 (DSM 4470) containing the PTA29 promoter (EP 344 029), ii) a FspI-HindIII fragment of pCO49 containing the sarnase-3'ocs fragment, and iii) the large EcoRI-HindIII fragment of pMC5-8, yielding the plasmid pCO50. Subsequently, the EcoRI-HindIII fragment (filled-in with Klenow) of pCO50, containing the chimeric PTA29-barnase-3'ocs gene, is cloned between the T-DNA borders of pGSJ260 (DSM 3606) by ligation with the large BamHI (filled-in with Klenow)-StuI fragment of pGSJ260. This yields the plasmid pTCO53, carrying a chimeric PTA29-R08/26 sarnase-3'ocs gene and a pnos-neo-3'ocs gene construct between the T-DNA border repeats.

Using an analagous procedure, a T-DNA vector is constructed carrying the R08/26 sarstar DNA of Examples 3, 4 and 5 under the control of the tapetum-specific PTA29 promoter, similar to the barstar-encoding vector, pTVE74, of EP 412 911.

The two resulting plasmid vectors are mobilized in Agrobacterium tumefaciens strain C58C1Rif(pGV2260) as described by Deblaere et al (1987) Meth. Enzymol. 153:277, yielding the sarnase DNA-containing strain C58C1Rif(pTCO1053) and the sarstar DNA-containing strain C58C1Rif(pTCO1054).

### Example 8: Plant transformations with chimeric genes of Example 7 containing the R08/26 sarnase or sarstar coding region under the control of the TA29 promoter

The Agrobacterium tumefaciens strains C58C1Rif (pTCO1053 and pTC01054) of Example 7 are used to transform lettuce (Lactuca sativa) according to the procedures described by Michelmore et al (1987) Plant Cell Reports 6:439 and tobacco (Nicotiana tabacum) as described for the barnase and RNase T1 chimeric constructs in EP 344 029 and EP 412 911. The resulting transformed plants are normal except for their flowers. In this regard, each plant containing the R08/26 sarnase DNA under the control of the TA29 tapetum-specific promoter expresses such DNA at least predominantly in its anthers and produces no normal pollen (i.e., the plant is male-sterile), and each plant containing the R08/26 sarstar DNA under the control of the TA29 promoter expresses such DNA at least predominantly in its anthers but produces normal pollen (i.e. the plant is a male-fertility restorer plant for the corresponding male-sterile plant of this Example). By crossing the male-sterile plants transformed with the R08/26 sarnase DNA with the corresponding male-fertility restorer plants transformed with the R08/26 sarstar DNA, fertile seed-bearing plants are obtained.

Needless to say, this invention is not limited to the transformation of any specific eucaryotic organism(s) or plant(s). The invention relates to any eucaryotic organism, particularly a plant, the nuclear genome of which can be transformed with the RNase DNA, preferably the sarnase DNA, under the control of the first promoter or with the RNase-inhibitor DNA, preferably the sarstar DNA, under the control of the second promoter. For example, this invention relates to plants such as corn, oilseed rape, wheat, rice, sunflower, sugarbeet, tomato, lettuce, peppers, sorghum, soybean, pea, alfalfa, grasses, clover, carrot, cabbages, leek, onion, tobacco, petunia, cacao and citrus trees.

Also this invention is not limited to the specific plasmids and vectors described in the foregoing Examples, but rather encompasses any plasmids and vectors containing the RNase DNA or RNase-inhibitor DNA.

Furthermore, this invention is not limited to the specific first and second promoters described in the foregoing Examples, i.e., the PTA29 promoter, but rather encompasses any DNA sequence encoding a first promoter capable of directing expression of a RNase DNA substantially selectively in cells of a specific organ and/or tissue of a eucaryotic organism, preferably a plant, particularly in the plant's flowers, seeds or embryos, or encoding a second promoter capable of directing expression of a RNase-inhibitor DNA at least in the cells of the organism where the RNase DNA is to be expressed.

In addition, this invention is not limited to the specific RNase and RNase-inhibitor DNAs described in the foregoing Examples but rather encompasses any equivalent DNA sequences encoding respectively: a RNase that would significantly disturb adversely the metabolism, functioning and/or development of eucaryotic cells, preferably plant cells, in which the equivalent RNase DNA is expressed under the control of the first promoter; and a RNase-inhibitor that, in a cell in which the equivalent RNase-inhibitor DNA is expressed under the control of the second promoter, would neutralize, block, offset, overcome or otherwise prevent the activity of the RNase encoded by the equivalent RNase DNA. In this regard, sarnase DNA and sarstar DNA sequences, other than those present naturally in S. aureofaciens strains and encoding natural sarnases and sarstars, can be used in accordance with this invention. Indeed, the natural sequences of the R08/26 sarnase and sarstar DNAs of this invention can be modified by: 1) replacing some codons with others that code either for the same amino acids or for other amino acids; and/or 2) deleting or adding some codons -- provided that such modifications do not substantially alter the properties, particularly the respective ribonuclease and ribonuclease-inhibiting properties, of the encoded R08/26 sarnases and sarstars. For example, the mature R08/26 sarnase of SEQ ID no. 1 can have its N-terminal end modified (e.g., by a methionine) to have proper expression of the sarnase within a eucaryotic organism.

Also this invention is not limited to the specific marker DNAs described in the foregoing Examples but rather encompasses any marker DNA encoding an RNA, protein or polypeptide that confers on at least a specific tissue or specific cells of the eucaryotic organism, particularly a plant, in which the marker DNA is expressed, a distinctive trait compared to such a specific tissue or specific cells in which the marker DNA is not expressed.

SEQUENCE LISTING

1. General Information

i)    APPLICANT : PLANT GENETIC SYSTEMS N.V.

ii)   TITLE OF INVENTION : A Novel Ribonuclease And Its
      Inhibitor

iii) NUMBER OF SEQUENCES : 3

iv)   CORRESPONDENCE ADDRESS :
           A. ADRDRESSEE : Plant Genetic Systems N.V.
           B. STREET : Plateaustraat 22
           C. POSTAL CODE AND CITY : 9000 Ghent
           D. COUNTRY : Belgium

v)    COMPUTER READABLE FORM :
           A. MEDIUM TYPE : 5.25 inch, double sided, high
           density 1.2 Mb floppy disk
           B. COMPUTER IBM PC/AT
           C. OPERATING SYSTEM : DOS version 3.3
           D. SOFTWARE : Wordperfect 5.1

vi)   CURRENT APPLICATION DATA : Not Available

vii) PRIOR APPLICATION DATA : Not Applicable

2. Sequence Description : SEQ ID NO. 1

SEQUENCE TYPE: DNA sequence with corresponding amino acid
sequence for a protein, R08/26 sarnase
SEQUENCE LENGTH: 977 bp and 163 translated amino acids

STRANDEDNESS: double stranded
TOPOLOGY: linear
MOLECULAR TYPE: genomic DNA

ORIGINAL SOURCE:
ORGANISM: Streptomyces aureofaciens R08/26

FEATURES: - nucleotide 149 to nucleotide 640 : coding
sequence of sarnase precursor, including a
signal sequence
- nucleotide 347 to nucleotide 640 : presumed
coding sequence of mature sarnase
- nucleotide 129 : cleavage site NarI
- nucleotide 221 : cleavage site KpnI
- nucleotide 253 : cleavage site StyI
- nucleotide 347 : cleavage site FspI (not
present in sequence, but introduced by site
directed mutagenesis of sequence 344-349 from
CCCGCC to TGCGCA).
- nucleotide 453 : cleavage site Tth111I
- nucleotide 524 : cleavage site BstEII
- nucleotide 588 : cleavage site ScaI
- nucleotide 643 : cleavage site Eco47III
- nucleotide 800 : cleavage site AvaI

PROPERTIES: The mature protein is a guanyl specific
ribonuclease

CGGCGGAACG GACAACGGCG TCCGTCCCGC CCGGCCGTAC GTCCTTGTGG 50
TGTCGGCGGG CGGTGCCCGT CGCGTGGTGC TCGTCGTGTG GTGTCCGTCA 100
TGTGTGGGAC ACGGCCGGCC GGCGCGGGCG CCTGCCAAGC TGGACCGC 148

ATG ACC AGA AGC AAG AGC CCG CTC GTC GTC GGG GCC GTC 187
MET Thr Arg Ser Lys Ser Pro Leu Val Val Gly Ala Val
-65 -60 -55

CTG ATC CTG GCC GTG CTC GCC GGG GTT GGG TAC CTG CTC 226
Leu Ile Leu Ala Val Leu Ala Gly Val Gly Tyr Leu Leu
-50 -45

GCC GGC AGG GGC GGC AGC ACC CAC CCC AAG GCC GCC GCG 265
Ala Gly Arg Gly Gly Ser Thr His Pro Lys Ala Ala Ala
-40 -35 -30

AGC TCC GCC GCC GCG GGC ACC TCG GCC CCC AAA TCC TCG 304
Ser Ser Ala Ala Ala Gly Thr Ser Ala Pro Lys Ser Ser
-25 -20 -15

GCT CCC AAG CCG TCC CCG CCC GCC GGC GGC GCC TGG ACG 343
Ala Pro Lys Pro Ser Pro Pro Ala Gly Gly Ala Trp Thr
-10 -5

CCC GCC GAC CCG GCG CTG GCC GAC GTC TGC CGC ACC AAG 382
Pro Ala Asp Pro Ala Leu Ala Asp Val Cys Arg Thr Lys
1               5                   10

CTG CCC AGC CAG GCC CAG GAC ACC CTC GCC CTG ATC GCC 421
Leu Pro Ser Gln Ala Gln Asp Thr Leu Ala Leu Ile Ala
    15                  20                  25

AAG AAC GGC CCC TAC CCG TAC AAC CGG GAC GGC GTC GTC 460
Lys Asn Gly Pro Tyr Pro Tyr Asn Arg Asp Gly Val Val
            30                  35

TTC GAG AAC CGC GAG AGC CGC CTG CCG AAG AAG GGC AAC 499
Phe Glu Asn Arg Glu Ser Arg Leu Pro Lys Lys Gly Asn
40                  45                  50

GGC TAC TAC CAC GAG TTC ACC GTG GTC ACC CCC GGC TCC 538
Gly Tyr Tyr His Glu Phe Thr Val Val Thr Pro Gly Ser
55 60

AAC GAC CGA GGC ACC CGA CGG GTC GTC ACC GGC GGC TAC 577
Asn Asp Arg Gly Thr Arg Arg Val Val Thr Gly Gly Tyr
65 70 75

GGC GAG CAG TAC TGG TCC CCG GAC CAC TAC GCG ACC TTC 616
Gly Glu Gln Tyr Trp Ser Pro Asp His Tyr Ala Thr Phe
    80                  85                  90

CAG GAG ATC GAC CCG CGC TGC TGA GCGCTNGCCT CACAGAAAAT 660
Gln Glu Ile Asp Pro Arg Cys .
95

ACTTTCCAAC CTGGAAAGGT CGTGGCACAG TGGAGCCACC GTCACTTTCC 710
GAGGGGGAAA CATGAGTGAC TCCGCTCCCA CCCCGCCGTC CGCCGCCGAG 760
GCACNGCCTG CCGACCGGGC CGGCGCGGCT GCCCGGCGCC CGGGCGAGCT 810
GCCCCGCTGG TACGGGCCGG CCTTCGCCTT CGCCTTCGCG CTCTACGGCC 860
TGGGGATCGG CCACGTCATC GAGACCGGGC AGATCGCCGT GATCGGCGGG 910
CTCGCNCTTC GCCGCCCTGA CCGGTGGCCT CGCGGCCTTC GCCATGCGCA 960
GCGACGGCAT CGTCCGC 977

3. Sequence Description : SEQ ID NO. 2

SEQUENCE TYPE: DNA sequence for an expression vector, pMac5-19.
SEQUENCE LENGTH: 4005 bp

STRANDEDNESS: double stranded
TOPOLOGY: circular
MOLECULAR TYPE: plasmid DNA replicable in E.coli

FEATURES: - nucleotide 3754 to nucleotide 3884 : tac
promoter
- nucleotide 3885 to nucleotide 3947 : PhoA signal
sequence
- nucleotide 3947 to nucleotide 3993 : part of
pUC19 polylinker (KpnI, SmaI, BamHI, XbaI, SalI,
BspMI, PstI, SphI, HindIII)

PROPERTIES: pMac5-19 is the basic expression vector from
which pMa5-19 and pMc5-19 can be derived. The
latter plasmids can be used for site directed
mutagenesis by a gapped duplex DNA method using
alternating selectable markers as described by
Stanssens et al (1989) NAR 17:4441. In pMc5-19
the G-residue at position 2238 of pMac5-19 is
replaced by a C which results in the insertion
of an amber codon in the bla-am gene. In pMa5-19
the G-residue at position 3409 of pMac5-19 is
replaced by a A which results in the insertion
of an amber codon in the cat gene.

AATTCACCTC GAAAGCAAGC TGATAAACCG ATACAATTAA AGGCTCCTTT 50
TGGAGCCTTT TTTTTTGGAG ATTTTCAACG TGAAAAAATT ATTATTCGCA 100
ATTCCAAGCT AATTCACCTC GAAAGCAAGC TGATAAACCG ATACAATTAA 150
AGGCTCCTTT TGGAGCCTTT TTTTTTGGAG ATTTTCAACG TGAAAAAATT 200
ATTATTCGCA ATTCCAAGCT CTGCCTCGCG CGTTTCGGTG ATGACGGTGA 250
AAACCTCTGA CACATGCAGC TCCCGGAGAC GGTCACAGCT TGTCTGTAAG 300
CGGATGCAGA TCACGCGCCC TGTAGCGGCG CATTAAGCGC GGCGGGTGTG 350
GTGGTTACGC GCAGCGTGAC CGCTACACTT GCCAGCGCCC TAGCGCCCGC 400
TCCTTTCGCT TTCTTCCCTT CCTTTCTCGC CACGTTCGCC GGCTTTCCCC 450
GTCAAGCTCT AAATCGGGGG CTCCCTTTAG GGTTCCGATT TAGTGCTTTA 500
CGGCACCTCG ACCCCAAAAA ACTTGATTAG GGTGATGGTT CACGTAGTGG 550
GCCATCGCCC TGATAGACGG TTTTTCGCCC TTTGACGTTG GAGTCCACGT 600
TCTTTAATAG TGGACTCTTG TTCCAAACTG GAACAACACT CAACCCTATC 650
TCGGTCTATT CTTTTGATTT ATAAGGGATT TTGCCGATTT CGGCCTATTG 700
GTTAAAAAAT GAGCTGATTT AACAAAAATT TAACGCGAAT TTTAACAAAA 750
TATTAACGTT TACAATTTGA TCTGCGCTCG GTCGTTCGGC TGCGGCGAGC 800
GGTATCAGCT CACTCAAAGG CGGTAATACG GTTATCCACA GAATCAGGGG 850
ATAACGCAGG AAAGAACATG TGAGCAAAAG GCCAGCAAAA GGCCAGGAAC 900
CGTAAAAAGG CCGCGTTGCT GGCGTTTTTC CATAGGCTCC GCCCCCCTGA 950

CGAGCATCAC AAAAATCGAC GCTCAAGTCA GAGGTGGCGA AACCCGACAG 1000
GACTATAAAG ATACCAGGCG TTTCCCCCTG GAAGCTCCCT CGTGCGCTCT 1050
CCTGTTCCGA CCCTGCCGCT TACCGGATAC CTGTCCGCCT TTCTCCCTTC 1100
GGGAAGCGTG GCGCTTTCTC AATGCTCACG CTGTAGGTAT CTCAGTTCGG 1150
TGTAGGTCGT TCGCTCCAAG CTGGGCTGTG TGCACGAACC CCCCGTTCAG 1200
CCCGACCGCT GCGCCTTATC CGGTAACTAT CGTCTTGAGT CCAACCCGGT 1250
AAGACACGAC TTATCGCCAC TGGCAGCAGC CACTGGTAAC AGGATTAGCA 1300
GAGCGAGGTA TGTAGGCGGT GCTACAGAGT TCTTGAAGTG GTGGCCTAAC 1350
TACGGCTACA CTAGAAGGAC AGTATTTGGT ATCTGCGCTC TGCTGAAGCC 1400
AGTTACCTTC GGAAAAAGAG TTGGTAGCTC TTGATCCGGC AAACAAACCA 1450
CCGCTGGTAG CGGTGGTTTT TTTGTTTGCA AGCAGCAGAT TACGCGCAGA 1500
AAAAAAGGAT CTCAAGAAGA TCCTTTGATC TTTTCTACGG GGTCTGACGC 1550
TCAGTGGAAC GAAAACTCAC GTTAAGGGAT TTTGGTCATG AGATTATCAA 1600
AAAGGATCTT CACCTAGATC CTTTTAAATT AAAAATGAAG TTTTAAATCA 1650
ATCTAAAGTA TATATGAGTA AACTTGGTCT GACAGTTACC AATGCTTAAT 1700
CAGTGAGGCA CCTATCTCAG CGATCTGTCT ATTTCGTTCA TCCATAGTTG 1750
CCTGACTCCC CGTCGTGTAG ATAACTACGA TACGGGAGGG CTTACCATCT 1800
GGCCCCAGTG CTGCAATGAT ACCGCGAGAC CCACGCTCAC CGGCTCCAGA 1850
TTTATCAGCA ATAAACCAGC CAGCCGGAAG GGCCGAGCGC AGAAGTGGTC 1900
CTGCAACTTT ATCCGCCTCC ATCCAGTCTA TTAATTGTTG CCGGGAAGCT 1950
AGAGTAAGTA GTTCGCCAGT TAATAGTTTG CGCAACGTTG TTGCCATTGC 2000
TGCAGGCATC GTGGTGTCAC GCTCGTCGTT TGGTATGGCT TCATTCAGCT 2050
CCGGTTCCCA ACGATCAAGG CGAGTTACAT GATCCCCCAT GTTGTGCAAA 2100
AAAGCGGTTA GCTCCTTCGG TCCTCCGATC GTTGTCAGAA GTAAGTTGGC 2150
CGCAGTGTTA TCACTCATGG TTATGGCAGC ACTGCATAAT TCTCTTACTG 2200
TCATGCCATC CGTAAGATGC TTTTCTGTGA CTGGTGAGTA CTCAACCAAG 2250
TCATTCTGAG AATAGTGTAT GCGGCGACCG AGTTGCTCTT GCCCGGCGTC 2300
AACACGGGAT AATACCGCGC CACATAGCAG AACTTTAAAA GTGCTCATCA 2350
TTGGAAAACG TTCTTCGGGG CGAAAACTCT CAAGGATCTT ACCGCTGTTG 2400
AGATCCAGTT CGATGTAACC CACTCGTGCA CCCAACTGAT CTTCAGCATC 2450
TTTTACTTTC ACCAGCGTTT CTGGGTGAGC AAAAACAGGA AGGCAAAATG 2500
CCGCAAAAAA GGGAATAAGG GCGACACGGA AATGTTGAAT ACTCATACTC 2550
TTCCTTTTTC AATATTATTG AAGCAGACAG TTTTATTGTT CATGATGATA 2600
TATTTTTATC TTGTGCAATG TAACATCAGA GATTTTGAGA CACAACGTGG 2650
CTTTGTTGAA TAAATCGAAC TTTTGCTGAG TTGACTCCCC GCGCGCGATG 2700
GGTCGAATTT GCTTTCGAAA AAAAAGCCCG CTCATTAGGC GGGCTAAAAA 2750
AAAGCCCGCT CATTAGGCGG GCTCGAATTT CTGCCATTCA TCCGCTTATT 2800
ATCACTTATT CAGGCGTAGC AACCAGGCGT TTAAGGGCAC CAATAACTGC 2850
CTTAAAAAAA TTACGCCCCG CCCTGCCACT CATCGCAGTA CTGTTGTAAT 2900
TCATTAAGCA TTCTGCCGAC ATGGAAGCCA TCACAGACGG CATGATGAAC 2950
CTGAATCGCC AGCGGCATCA GCACCTTGTC GCCTTGCGTA TAATATTTGC 3000
CCATAGTGAA AACGGGGGCG AAGAAGTTGT CCATATTCGC CACGTTTAAA 3050
TCAAAACTGG TGAAACTCAC CCAGGGATTG GCTGAGACGA AAAACATATT 3100
CTCAATAAAC CCTTTAGGGA AATAGGCCAG GTTTTCACCG TAACACGCCA 3150
CATCTTGCGA ATATATGTGT AGAAACTGCC GGAAATCGTC GTGGTATTCA 3200
CTCCAGAGCG ATGAAAACGT TTCAGTTTGC TCATGGAAAA CGGTGTAACA 3250
AGGGTGAACA CTATCCCATA TCACCAGCTC ACCGTCTTTC ATTGCCATAC 3300
GAAATTCCGG ATGAGCATTC ATCAGGCGGG CAAGAATGTG AATAAAGGCC 3350
GGATAAAACT TGTGCTTATT TTTCTTTACG GTCTTTAAAA AGGCCGTAAT 3400
ATCCAGCTGA ACGGTCTGGT TATAGGTACA TTGAGCAACT GACTGAAATG 3450

CCTCAAAATG TTCTTTACGA TGCCATTGGG ATATATCAAC GGTGGTATAT 3500
CCAGTGATTT TTTTCTCCAT TTTAGCTTCC TTAGCTCCTG AAAATCTCGA 3550
TAACTCAAAA AATACGCCCG GTAGTGATCT TATTTCATTA TGGTGAAAGT 3600
TGGAACCTCT TACGTGCCGA TCAACGTCTC ATTTTCGCCA AAAGTTGGCC 3650
CAGGGCTTCC CGGTATCAAC AGGGACACCA GGATTTATTT ATTCTGCGAA 3700
GTGATCTTCC GTCACAGGTA TTTATTCGAA GACGAAAGGG CATCGCGCGC 3750
GGGGAATTCG AGCTCGAGCT TACTCCCCAT CCCCCTGTTG ACAATTAATC 3800
ATCGGCTCGT ATAATGTGTG GAATTGTGAG CGGATAACAA TTTCACACAG 3850
GAAACAGGAT CCGCGGATCC GTGGAGAAAA TAAAATGAAA CAAAGCACTA 3900
TTGCACTGGC ACTCTTACCG TTACTGTTTA CCCCTGTGAC AAAAGCGGTA 3950
CCCGGGGATC CTCTAGAGTC GACCTGCAGG CATGCAAGCT TGGTCTAGAG 4000
GTCGA 4005

4. Sequence Description : SEQ ID NO. 3

SEQUENCE TYPE: DNA sequence of a plasmid, pTE4
SEQUENCE LENGTH: 7049 bp

STRANDEDNESS: double stranded
TOPOLOGY: circular
MOLECULAR TYPE: plasmid DNA replicable in E.coli

FEATURES: - nucleotide (nt) 1 - nt 191 : pUC18 with a 242 bp NdeI-
BamHI deletion
- nt 192 to nt 681 : Pnos
- nt 682 - nt 2490 : gus coding sequence
- nt 2491 - nt 2792 : 3'nos
- nt 2793 - nt 3637 : 35S promoter
- nt 3638 - nt 3973 : barnase coding sequence
- nt 3974 - nt 4802 : 3'ocs
- nt 4803 - end : pUC18

PROPERTIES: pTE4 can be used for elctroporation of plant cells to
evaluate the transient expression of the Pnos-gus-3'ocs and 35S-
barnase-3'ocs chimaeric genes. The cytotoxic effect of the barnase
gene can be monitored by the expression of GUS.

TCGCGCGTTT CGGTGATGAC GGTGAAAACC TCTGACACAT GCAGCTCCCG 50
GAGACGGTCA CAGCTTGTCT GTAAGCGGAT GCCGGGAGCA GACAAGCCCG 100
TCAGGGCGCG TCAGCGGGTG TTGGCGGGTG TCGGGGCTGG CTTAACTATG 150
CGGCATCAGA GCAGATTGTA CTGAGAGTGC ACCATAGATC CGTCAATTCC 200
GATTCATTAA TGCAGCTGGC ACGACAGGTT TCCCGACTGG AAAGCGGGCA 250
GTGAGCGCAA CGCAATTAAT GTGAGTTAGC TCACTCATTA GGCACCCCAG 300
GCTTTACACT TTATGCTTCC GGCTCGTATA ATGTGTGGAA TTGTGAGCGG 350
ATAACAATTT CACACAGGAA ACAGGATCAT GAGCGGAGAA TTAAGGGAGT 400
CACGTTATGA CCCCCGCCGA TGACGCGGGA CAAGCCGTTT TACGTTTGGA 450
ACTGACAGAA CCGCAACGAT TGAAGGAGCC ACTCAGCCGC GGGTTTCTGG 500
AGTTTAATGA GCTAAGCACA TACGTCAGAA ACCATTATTG CGCGTTCAAA 550
AGTCGCCTAA GGTCACTATC AGCTAGCAAA TATTTCTTGT CAAAAATGCT 600
CCACTGACGT TCCATAAATT CCCCTCGGTA TCCAATTAGA GTCTCATATT 650
CACTCTCAAT CAAAGATCCG GCCCATGATC CATGGTCCGT CCTGTAGAAA 700
CCCCAACCCG TGAAATCAAA AAACTCGACG GCCTGTGGGC ATTCAGTCTG 750
GATCGCGAAA ACTGTGGAAT TGATCAGCGT TGGTGGGAAA GCGCGTTACA 800
AGAAAGCCGG GCAATTGCTG TGCCAGGCAG TTTTAACGAT CAGTTCGCCG 850
ATGCAGATAT TCGTAATTAT GCGGGCAACG TCTGGTATCA GCGCGAAGTC 900
TTTATACCGA AAGGTTGGGC AGGCCAGCGT ATCGTGCTGC GTTTCGATGC 950
GGTCACTCAT TACGGCAAAG TGTGGGTCAA TAATCAGGAA GTGATGGAGC 1000
ATCAGGGCGG CTATACGCCA TTTGAAGCCG ATGTCACGCC GTATGTTATT 1050
GCCGGGAAAA GTGTACGTAT CACCGTTTGT GTGAACAACG AACTGAACTG 1100
GCAGACTATC CCGCCGGGAA TGGTGATTAC CGACGAAAAC GGCAAGAAAA 1150
AGCAGTCTTA CTTCCATGAT TTCTTTAACT ATGCCGGAAT CCATCGCAGC 1200
GTAATGCTCT ACACCACGCC GAACACCTGG GTGGACGATA TCACCGTGGT 1250

GACGCATGTC GCGCAAGACT GTAACCACGC GTCTGTTGAC TGGCAGGTGG 1300
TGGCCAATGG TGATGTCAGC GTTGAACTGC GTGATGCGGA TCAACAGGTG 1350
GTTGCAACTG GACAAGGCAC TAGCGGGACT TTGCAAGTGG TGAATCCGCA 1400
CCTCTGGCAA CCGGGTGAAG GTTATCTCTA TGAACTGTGC GTCACAGCCA 1450
AAAGCCAGAC AGAGTGTGAT ATCTACCCGC TTCGCGTCGG CATCCGGTCA 1500
GTGGCAGTGA AGGGCGAACA GTTCCTGATT AACCACAAAC CGTTCTACTT 1550
TACTGGCTTT GGTCGTCATG AAGATGCGGA CTTACGTGGC AAAGGATTCG 1600
ATAACGTGCT GATGGTGCAC GACCACGCAT TAATGGACTG GATTGGGGCC 1650
AACTCCTACC GTACCTCGCA TTACCCTTAC GCTGAAGAGA TGCTCGACTG 1700
GGCAGATGAA CATGGCATCG TGGTGATTGA TGAAACTGCT GCTGTCGGCT 1750
TTAACCTCTC TTTAGGCATT GGTTTCGAAG CGGGCAACAA GCCGAAAGAA 1800
CTGTACAGCG AAGAGGCAGT CAACGGGGAA ACTCAGCAAG CGCACTTACA 1850
GGCGATTAAA GAGCTGATAG CGCGTGACAA AAACCACCCA AGCGTGGTGA 1900
TGTGGAGTAT TGCCAACGAA CCGGATACCC GTCCGCAAGT GCACGGGAAT 1950
ATTTCGCCAC TGGCGGAAGC AACGCGTAAA CTCGACCCGA CGCGTCCGAT 2000
CACCTGCGTC AATGTAATGT TCTGCGACGC TCACACCGAT ACCATCAGCG 2050
ATCTCTTTGA TGTGCTGTGC CTGAACCGTT ATTACGGATG GTATGTCCAA 2100
AGCGGCGATT TGGAAACGGC AGAGAAGGTA CTGGAAAAAG AACTTCTGGC 2150
CTGGCAGGAG AAACTGCATC AGCCGATTAT CATCACCGAA TACGGCGTGG 2200
ATACGTTAGC CGGGCTGCAC TCAATGTACA CCGACATGTG GAGTGAAGAG 2250
TATCAGTGTG CATGGCTGGA TATGTATCAC CGCGTCTTTG ATCGCGTCAG 2300
CGCCGTCGTC GGTGAACAGG TATGGAATTT CGCCGATTTT GCGACCTCGC 2350
AAGGCATATT GCGCGTTGGC GGTAACAAGA AAGGGATCTT CACTCGCGAC 2400
CGCAAACCGA AGTCGGCGGC TTTTCTGCTG CAAAAACGCT GGACTGGCAT 2450
GAACTTCGGT GAAAAACCGC AGCAGGGAGG CAAACAATGA XXXXXXGAAT 2500
TGATCCGAAG CAGATCGTTC AAACATTTGG CAATAAAGTT TCTTAAGATT 2550
GAATCCTGTT GCCGGTCTTG CGATGATTAT CATATAATTT CTGTTGAATT 2600
ACGTTAAGCA TGTAATAATT AACATGTAAT GCATGACGTT ATTTATGAGA 2650
TGGGTTTTTA TGATTAGAGT CCCGCAATTA TACATTTAAT ACGCGATAGA 2700
AAACAAAATA TAGCGCGCAA ACTAGGATAA ATTATCGCGC GCGGTGTCAT 2750
CTATGTTACT AGATCGGGAA GATCCTCTAG AGTCGACCTG CAGGCATGCA 2800
AGCTCCTACG CAGCAGGTCT CATCAAGACG ATCTACCCGA GTAACAATCT 2850
CCAGGAGATC AAATACCTTC CCAAGAAGGT TAAAGATGCA GTCAAAAGAT 2900
TCAGGACTAA TTGCATCAAG AACACAGAGA AAGACATATT TCTCAAGATC 2950
AGAAGTACTA TTCCAGTATG GACGATTCAA GGCTTGCTTC ATAAACCAAG 3000
GCAAGTAATA GAGATTGGAG TCTCTAAAAA GGTAGTTCCT ACTGAATCTA 3050
AGGCCATGCA TGGAGTCTAA GATTCAAATC GAGGATCTAA CAGAACTCGC 3100
CGTGAAGACT GGCGAACAGT TCATACAGAG TCTTTTACGA CTCAATGACA 3150
AGAAGAAAAT CTTCGTCAAC ATGGTGGAGC ACGACACTCT GGTCTACTCC 3200
AAAAATGTCA AAGATACAGT CTCAGAAGAC CAAAGGGCTA TTGAGACTTT 3250
TCAACAAAGG ATAATTTCGG GAAACCTCCT CGGATTCCAT TGCCCAGCTA 3300
TCTGTCACTT CATCGAAAGG ACAGTAGAAA AGGAAGGTGG CTCCTACAAA 3350
TGCCATCATT GCGATAAAGG AAAGGCTATC ATTCAAGATG CCTCTGCCGA 3400
CAGTGGTCCC AAAGATGGAC CCCCACCCAC GAGGAGCATC GTGGAAAAAG 3450
AAGACGTTCC AACCACGTCT TCAAAGCAAG TGGATTGATG TGACATCTCC 3500
ACTGACGTAA GGGATGACGC ACAATCCCAC TATCCTTCGC AAGACCCTTC 3550
CTCTATATAA GGAAGTTCAT TTCATTTGGA GAGGACACGC TGAAATCACC 3600
AGTCTCTCTC TATAAATCTA TCTCTCTCTC TATAACCATG GTACCGGTTA 3650
TCAACACGTT TGACGGGGTT GCGGATTATC TTCAGACATA TCATAAGCTA 3700
CCTGATAATT ACATTACAAA ATCAGAAGCA CAAGCCCTCG GCTGGGTGGC 3750

ATCAAAAGGG AACCTTGCAG ACGTCGCTCC GGGGAAAAGC ATCGGCGGAG 3800
ACATCTTCTC AAACAGGGAA GGCAAACTCC CGGGCAAAAG CGGACGAACA 3850
TGGCGTGAAG CGGATATTAA CTATACATCA GGCTTCAGAA ATTCAGACCG 3900
GATTCTTTAC TCAAGCGACT GGCTGATTTA CAAAACAACG GACCATTATC 3950
AGACCTTTAC AAAAATCAGA TAACGAAAAA AACGGCTTCC TGCGGAGGCC 4000
GTTTTTTTCA GCTTTACATA AAGTGTGTAA TAAATTTTTC TTCAAACTCT 4050
GATCGGTCAA TTTCACTTTC CGGXXXXCTC TAGAGTCCTG CTTTAATGAG 4100
ATATGCGAGA CGCCTATGAT CGCATGATAT TTGCTTTCAA TTCTGTTGTG 4150
CACGTTGTAA AAAACCTGAG CATGTGTAGC TCAGATCCTT ACCGCCGGTT 4200
TCGGTTCATT CTAATGAATA TATCACCCGT TACTATCGTA TTTTTATGAA 4250
TAATATTCTC CGTTCAATTT ACTGATTGTA CCCTACTACT TATATGTACA 4300
ATATTAAAAT GAAAACAATA TATTGTGCTG AATAGGTTTA TAGCGACATC 4350
TATGATAGAG CGCCACAATA ACAAACAATT GCGTTTTATT ATTACAAATC 4400
CAATTTTAAA AAAAGCGGCA GAACCGGTCA AACCTAAAAG ACTGATTACA 4450
TAAATCTTAT TCAAATTTCA AAAGGCCCCA GGGGCTAGTA TCTACGACAC 4500
ACCGAGCGGC GAACTAATAA CGTTCACTGA AGGGAACTCC GGTTCCCCGC 4550
CGGCGCGCAT GGGTGAGATT CCTTGAAGTT GAGTATTGGC CGTCCGCTCT 4600
ACCGAAAGTT ACGGGCACCA TTCAACCCGG TCCAGCACGG CGGCCGGGTA 4650
ACCGACTTGC TGCCCCGAGA ATTATGCAGC ATTTTTTTGG TGTATGTGGG 4700
CCCCAAATGA AGTGCAGGTC AAACCTTGAC AGTGACGACA AATCGTTGGG 4750
CGGGTCCAGG GCGAATTTTG CGACAACATG TCGAGGCTCA GCAGGACCTG 4800
CAGGCATGCA AGCTTGGCGT AATCATGGTC ATAGCTGTTT CCTGTGTGAA 4850
ATTGTTATCC GCTCACAATT CCACACAACA TACGAGCCGG AAGCATAAAG 4900
TGTAAAGCCT GGGGTGCCTA ATGAGTGAGC TAACTCACAT TAATTGCGTT 4950
GCGCTCACTG CCCGCTTTCC AGTCGGGAAA CCTGTCGTGC CAGCTGCATT 5000
AATGAATCGG CCAACGCGCG GGGAGAGGCG GTTTGCGTAT TGGGCGCTCT 5050
TCCGCTTCCT CGCTCACTGA CTCGCTGCGC TCGGTCGTTC GGCTGCGGCG 5100
AGCGGTATCA GCTCACTCAA AGGCGGTAAT ACGGTTATCC ACAGAATCAG 5150
GGGATAACGC AGGAAAGAAC ATGTGAGCAA AAGGCCAGCA AAAGGCCAGG 5200
AACCGTAAAA AGGCCGCGTT GCTGGCGTTT TTCCATAGGC TCCGCCCCCC 5250
TGACGAGCAT CACAAAAATC GACGCTCAAG TCAGAGGTGG CGAAACCCGA 5300
CAGGACTATA AAGATACCAG GCGTTTCCCC CTGGAAGCTC CCTCGTGCGC 5350
TCTCCTGTTC CGACCCTGCC GCTTACCGGA TACCTGTCCG CCTTTCTCCC 5400
TTCGGGAAGC GTGGCGCTTT CTCAATGCTC ACGCTGTAGG TATCTCAGTT 5450
CGGTGTAGGT CGTTCGCTCC AAGCTGGGCT GTGTGCACGA ACCCCCCGTT 5500
CAGCCCGACC GCTGCGCCTT ATCCGGTAAC TATCGTCTTG AGTCCAACCC 5550
GGTAAGACAC GACTTATCGC CACTGGCAGC AGCCACTGGT AACAGGATTA 5600
GCAGAGCGAG GTATGTAGGC GGTGCTACAG AGTTCTTGAA GTGGTGGCCT 5650
AACTACGGCT ACACTAGAAG GACAGTATTT GGTATCTGCG CTCTGCTGAA 5700
GCCAGTTACC TTCGGAAAAA GAGTTGGTAG CTCTTGATCC GGCAAACAAA 5750
CCACCGCTGG TAGCGGTGGT TTTTTTGTTT GCAAGCAGCA GATTACGCGC 5800
AGAAAAAAAG GATCTCAAGA AGATCCTTTG ATCTTTTCTA CGGGGTCTGA 5850
CGCTCAGTGG AACGAAAACT CACGTTAAGG GATTTTGGTC ATGAGATTAT 5900
CAAAAAGGAT CTTCACCTAG ATCCTTTTAA ATTAAAAATG AAGTTTTAAA 5950
TCAATCTAAA GTATATATGA GTAAACTTGG TCTGACAGTT ACCAATGCTT 6000
AATCAGTGAG GCACCTATCT CAGCGATCTG TCTATTTCGT TCATCCATAG 6050
TTGCCTGACT CCCCGTCGTG TAGATAACTA CGATACGGGA GGGCTTACCA 6100
TCTGGCCCCA GTGCTGCAAT GATACCGCGA GACCCACGCT CACCGGCTCC 6150
AGATTTATCA GCAATAAACC AGCCAGCCGG AAGGGCCGAG CGCAGAAGTG 6200
GTCCTGCAAC TTTATCCGCC TCCATCCAGT CTATTAATTG TTGCCGGGAA 6250

GCTAGAGTAA GTAGTTCGCC AGTTAATAGT TTGCGCAACG TTGTTGCCAT 6300
TGCTACAGGC ATCGTGGTGT CACGCTCGTC GTTTGGTATG GCTTCATTCA 6350
GCTCCGGTTC CCAACGATCA AGGCGAGTTA CATGATCCCC CATGTTGTGC 6400
AAAAAAGCGG TTAGCTCCTT CGGTCCTCCG ATCGTTGTCA GAAGTAAGTT 6450
GGCCGCAGTG TTATCACTCA TGGTTATGGC AGCACTGCAT AATTCTCTTA 6500
CTGTCATGCC ATCCGTAAGA TGCTTTTCTG TGACTGGTGA GTACTCAACC 6550
AAGTCATTCT GAGAATAGTG TATGCGGCGA CCGAGTTGCT CTTGCCCGGC 6600
GTCAATACGG GATAATACCG CGCCACATAG CAGAACTTTA AAAGTGCTCA 6650
TCATTGGAAA ACGTTCTTCG GGGCGAAAAC TCTCAAGGAT CTTACCGCTG 6700
TTGAGATCCA GTTCGATGTA ACCCACTCGT GCACCCAACT GATCTTCAGC 6750
ATCTTTTACT TTCACCAGCG TTTCTGGGTG AGCAAAAACA GGAAGGCAAA 6800
ATGCCGCAAA AAAGGGAATA AGGGCGACAC GGAAATGTTG AATACTCATA 6850
CTCTTCCTTT TTCAATATTA TTGAAGCATT TATCAGGGTT ATTGTCTCAT 6900
GAGCGGATAC ATATTTGAAT GTATTTAGAA AAATAAACAA ATAGGGGTTC 6950
CGCGCACATT TCCCCGAAAA GTGCCACCTG ACGTCTAAGA AACCATTATT 7000
ATCATGACAT TAACCTATAA AAATAGGCGT ATCACGAGGC CCTTTCGTC 7049

## Claims

1. A method of significantly disrupting the metabolism, functioning and/or development of selected cells, especially cells of a selected tissue and/or organ, of an eucaryotic organism, particularly a plant, by inserting into each cell of said organism, preferably by stably integrating into the nuclear genome of each cell of said organism, a first chimaeric gene comprising:
i) a Rnase DNA coding for a protein which has RNase activity and which contains only one intramolecular disulfide bridge; said RNase DNA preferably being a sarnase DNA, particularly an R08/26 sarnase DNA; and
ii) a first promoter capable of directing the expression of said Rnase DNA substantially only in said selected cells of said organism; said Rnase DNA being in the same transcriptional unit as, and under the control of, said first promoter.

2. The method of claim 1 in which said Rnase DNA is obtainable from a Streptomyces aureofaciens, preferably strain R08/26, particularly the RNAse DNA which has the nucleotide sequence of SEQ ID no. 1 or codes for the RNase with the amino acid sequence of SEQ ID no. 1.

3. The method of claim 1 or 2 in which said organism is a plant and in which said first promoter is capable of directing expression of said Rnase DNA selectively in the cells of a male reproductive organ of said plant, particularly the anther, pollen or filament cells, quite particularly in the anther epidermal cells or tapetum cells, or in which said promoter is capable of directing expression of said Rnase DNA selectively in the cells of a female reproductive organs or of seeds or of embryos of said plant, particularly in style, stigma, ovule, septum, seed coat, endosperm, embryo axis or embryo cotyledon cells.

4. The method of claim 1 or 2 in which said organism is a plant and said first promoter is induced by the presence of a nematode, particularly in plant cells of fixed feeding sites.

5. A method to prevent or diminish the disruption of normal metabolism, functioning or development of selected cells of a eucaryotic organism, particularly a plant, containing in each of its cells the first chimaeric gene of any one of claims 1 to 4, by inserting into each cell of said organism, preferably by stably integrating into the nuclear genome of each cell of said organism, a second chimaeric gene comprising:
i) a RNase-inhibitor DNA coding for a protein that inhibits the RNAse activity encoded by the first chimaeric gene; said RNase-inhibitor DNA preferably being a sarstar DNA, particularly a R08/26 sarstar DNA; and
ii) a second promoter capable of directing the expression of said RNase DNA at least in said selected cells of said organism; said RNase-inhibitor DNA being in the same transcriptional unit as, and under the control of, said second promoter.

6. The method of claim 5 in which said RNase-inhibitor DNA is obtainable from a S. aureofaciens, preferably strain R08/26

7. The method of claim 5 or 6 in which: said organism is a plant; said first promoter is the promoter of claim 3; and said second promoter is the same as said first promoter or is a constitutive promoter, such as a 35S promoter.

8. A method to produce hybrid seed comprising crossing a plant of a species containing, in each of its cells, said first chimaeric gene of claim 3 with a non-transformed plant of said species or with a plant of said species containing, in each of its cells, said second chimaeric gene of claim 7.

9. A cell of an eucaryotic organism, particularly a plant, the nuclear genome of which is transformed with said first chimaeric gene, said second chimaeric gene, or both of any one of claims 1 to 7.

10. A plant or plant culture consisting of the cells of claim 9.

11. A seed of the plant of claim 10.

12. A R08/26 sarnase DNA that is obtainable from Streptomyces aureofaciens strain R08/26 (DSM 6691) and that preferably has the nucleotide sequence of SEQ ID no. 1 or codes for the RNase with the amino acid sequence of SEQ ID no. 1.

13. A R08/26 sarnase, preferably having the amino acid sequence of SEQ ID no. 1.

14. A R08/26 sarstar DNA that is obtainable from Streptomyces aureofaciens strain R08/26 (DSM 6691) and codes for a protein that is capable of inhibiting the RNase activity of the R08/26 sarnase of claim 13.

15. A R08/26 sarstar that is obtainable from Streptomyces aureofaciens strain R08/26 and is capable of inhibiting the Rnase activity of the R08/26 sarnase of claim 13.

16. A plasmid, such as a Ti-plasmid, comprising said first chimaeric gene of any one of claims 1 to 4 or said second chimaeric gene of any one of claims 5 to 7.

17. The Streptomyces aureofaciens strain R08/26 (DSM 6691).
